# EUROPEAN PATENT SPECIFICATION

(11) **EP 3 323 434 B1**
(45) Date of publication and mention of the grant of the patent: **15.01.2020**
(21) Application number: 16199197.1
(22) Date of filing: 16.11.2016
(51) Int. Cl.: A61L 9/03

(54) **DISPENSING DEVICE HAVING AN ANTI-DRIPPING SYSTEM**
AUSGABEVORRICHTUNG MIT EINEM TROPFSCHUTZSYSTEM
DISPOSITIF DE DISTRIBUTION AYANT UN SYSTÈME ANTI-GOUTTE

(43) Date of publication of application: 23.05.2018
(73) Proprietor: Eurvest S.A., 1400 Nivelles (BE)
(72) Inventor: DMITRUK, Aleksandra, 20-461 Lublin (PL); LUCIANI, Alain, 13390 Auriol (FR); SLAWEK, Monika, 20-553 Lublin (PL)
(74) Representative: August Debouzy

(56) References cited:
- EP-A1- 1 792 633
- EP-A1- 2 545 942
- WO-A1-2011/088097
- WO-A1-2011/120073
- JP-A- H09 248 330
- US-A1- 2007 023 541

## Description

### FIELD OF THE INVENTION

The proposed device relates to a dispensing device for the release of volatile compositions. More particularly the instant invention deals with means of dispersing a fragrance into a closed atmosphere, typically a house, the invention further having an anti-dripping system.

### TECHNICAL BACKGROUND

EP1792633 discloses a device for diffusing volatile substances comprising two containers to hold respective liquids with respective volatile substances. Each container has at least one aperture closed by a cap. The device is configured so that it can be located on a supporting surface with the containers vertically confronting, so that one of the containers is selectively in an upper position or in a lower position with respect to the other container. The container which is in the upper position emanates the volatile substance which it contains, and the other one does not emanate the volatile substance which it contains.

Volatile compositions such as fragrances can be released into the atmosphere. Some dispensing devices release the composition by convection, or passive diffusion.

For example, it is known in the art to fill a closed basin with a volatile liquid. The basin comprises one through slot to allow one rigid strip to dip into the volatile liquid. While one of the extremities of the strip is in contact with the liquid of the basin, the other one is in contact with the external environment. Typically, the composition travels along the strip by capillarity to be released in the environment. Similar types of dispenser are disclosed in US 2009/0101729.

These devices however comprise several drawbacks as their release capabilities is limited. For example, the strip is subjected to a clogging effect: the prolonged use of the dispensing material leads to an internal degradation of the material which eventually impacts its capability to release the composition into the environment. Therefore, it is usually necessary to change the dispensing material although the user is not completely certain of the best time to change it. The user further needs to touch the used material which leads to chemical contamination.

Devices of the type "plug and smell" have also been disclosed in the art. These solutions involve a volatile liquid reservoir from which a cellulosic wick is upwardly and outwardly extending. The device uses several seals and sealing surfaces between the cellulosic wick and the reservoir, providing enhanced spill resistance. This type of dispenser is disclosed for example in WO 2012/033526. However, the spill resistance features disclosed are costly and industrially complicated to implement as they require precision in the manufacturing.

Other dispensers are electrically powered to actively enhance the delivery of the volatile composition, i.e. by heating the composition contained in the reservoir.

However, these devices do not offer the same flexibility to the user compared to passive dispensers because the user needs to plug the dispenser directly to the electrical outlet. Thus, the user is not free to choose any desired location to place its device within a room. Furthermore, these volatile liquid dispensing devices are usually intended to operate over an extended period of time and occupy valuable electrical outlets once in service.

Further, this type of solutions still has the drawback of not preventing from any leakages at a good cost and easily manufactured if the device is rotated. This leakage of product, causing the decrease of the amount of liquid contained in the reservoir, lead to a diminution of the efficiency of the device. The rate of dispersion is also decreased in case of leakage.

Another drawback of the known active devices derives from the finding that some users prefer to hide dispenser, e.g. behind an object, while it remains noticeable on an electrical outlet.

Thus, there is still a need for a volatile liquid dispensing device which overcomes all the above mentioned drawbacks while achieving an increased release efficacy.

### SUMMARY OF THE INVENTION

This has been achieved with the instant invention.

It is notably proposed a dispensing device comprising :
- a basin adapted to contain a volatile liquid to be diffused into the atmosphere, the basin containing an access opening from the outside to the volatile liquid contained therein;
- a dispensing material adapted to be in fluid communication with the basin through the access opening to allow the volatile liquid to diffuse into the atmosphere;
the basin including an anti-dripping system comprising an absorbing element, the absorbing element being disposed in the basin by covering the access opening.

According to preferred embodiments, the proposed dispensing device may also comprises at least one of the following additional features :
- the absorbing element fills the basin;
- the absorbing element extends up to the access opening of the basin;
- the absorbing element is made of a porous material;
- the porous material is a foam material, preferably a sponge, more preferably a synthetic or cellulosic sponge;
- the dispensing device comprises the volatile liquid inside the basin, the volatile liquid being a fragrance;
- the dispensing device comprises a base including the basin, the base further comprising a heating element for heating the basin and, if necessary, the liquid inside the basin,, the heating element being preferably at least one light bulb or at least one resistor;
- the basin is an intermediate basin and the dispensing device comprises a filling port adapted to receive a reservoir having a rupturable seal and filled with volatile liquid, the filling port comprising a piercer for rupturing the rupturable seal upon engagement of the reservoir into the filling port, the dispensing device preferably including the reservoir;
- the dispensing device comprises a cartridge including the dispensing material and the reservoir, the cartridge further comprising a coupling element holding together the reservoir and the dispensing material;
- the access opening is in the form of a through slot extending from one side to the other of the filling port;
- the dispensing device comprises a display housing, preferably made of paper, and being able to be placed onto the dispensing device in order to surround and mask, at least partially, the dispensing device.

According to the present invention, there also is provided a process for dispensing volatile liquid comprising the steps of:
- providing the proposed device,
- plugging the reservoir into the filling port of the device, thereby making the volatile liquid therein into fluid communication with the basin of the device;
- placing the dispensing material of the device into fluid communication with the basin through the access opening.

According to preferred embodiments, the proposed process may also comprises at least one of the following additional features :
- the dispensing material is placed into fluid communication with the basin by contacting the absorbing element;
- the reservoir plugged into the filling port is compressing the absorbing element into the basin;
- the dispensing material is placed into fluid communication with the basin by plugging the reservoir into the filling port of the device.

### FIGURES

Figure 1 illustrates the overall view of an appropriate base that can be used in a dispenser according to the invention
Figure 2 illustrates a schematic top view of the base according to Figure 1.
Figure 3 illustrates a schematic top view of the base according to an embodiment in alternative to the embodiment of Figure 1
Figure 4 illustrates a cross section view along the axe B-B of the base according to Figures 2 and 3 without absorbing element in the basin.
Figure 5 and 6 illustrate a reservoir together with a dispensing material forming a cartridge that may be used in a dispenser according to two different embodiments of the invention.
Figure 7 and 8 illustrate respectively a front view and a cross-section view of a cartridge according to the embodiment of Figure 5.
Figure 9 illustrates the cross section view of Figure 4, with the absorbing element disposed in the basin.
Figure 10 illustrates the cross section view of Figure 9 with the cartridge of figures 5, 7 and 8.
Figure 11 is an exploded representation of an assembled dispenser according to the embodiment of figure 1.
Figure 12 illustrates a representation of an assembled dispenser according to the embodiment of Figure 10

### DESCRIPTION OF EMBODIMENTS OF THE INVENTION

The proposed device relates to a passive dispensing device or apparatus for the delivery of a volatile liquid into the atmosphere. The proposed device notably relates to slow release volatile liquid dispensing device. Particularly, the device according to the invention is intended to provide fragrance to the environment.

Within the meaning of the proposed device, the terms "dispenser" and "dispensing device" are used indifferently.

One element of the proposed dispenser is the basin. The basin typically corresponds to a recipient which is adapted to contain a volatile liquid therein. The following description is relative to the dispenser comprising volatile liquid inside the basin. However this description equally applies to the same dispenser before or after having received any volatile liquid in the basin.

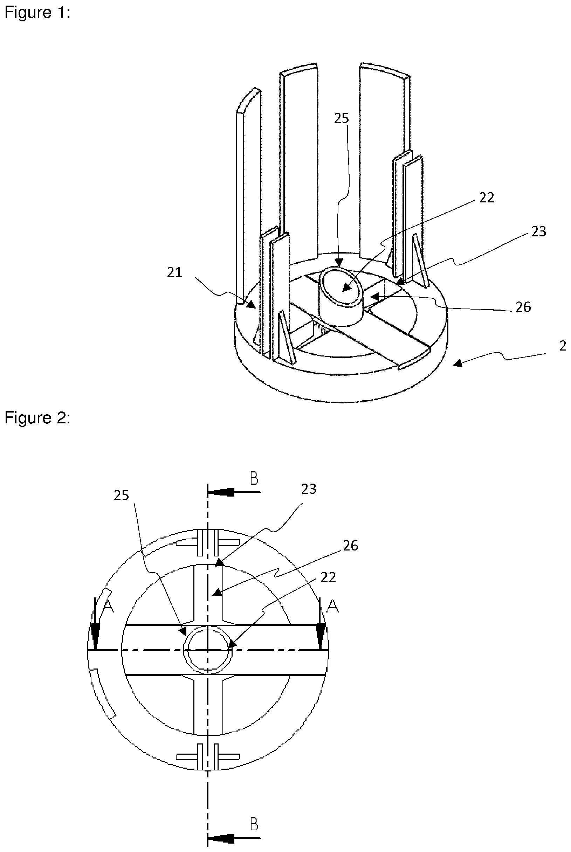

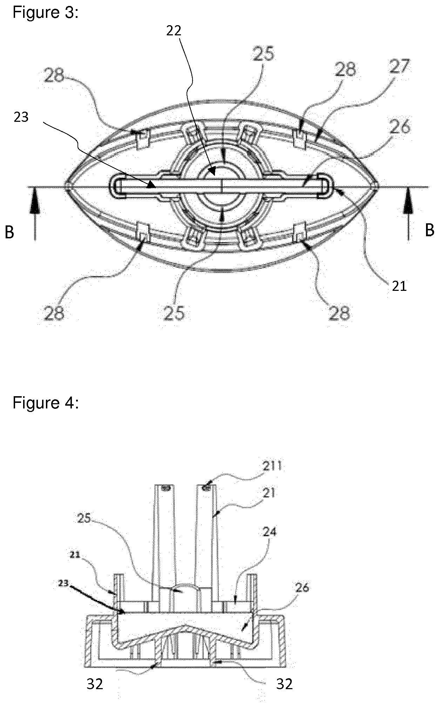

Figure 1 shows a base 2 that can be used in a dispenser according to the invention. Figure 2 shows a top view of the base according to Figure 1. Figure 3 shows a top view of the base according to an embodiment in alternative to the embodiment of Figure 1. Accordingly the base may be circular, oval, or of any other appropriate shape.

Figure 4 shows a cross section view along the axe B-B of the base according to Figures 2 and 3.

This base 2 included the basin 26. Appropriate basins are made of an impermeable material, for example of plastic, which are suited to contain a volatile liquid therein.

Within the meaning of the proposed device, the term "volatile liquid" means any liquid composition which evaporates at ambient temperature. The volatile liquid is notably here a fragrance.

Appropriate dimensions for a basin may be: a length comprised within the range of 30 to 70 mm, typically within the range of 40 to 60 mm such as 50 mm, a width comprised within the range of 3 to 15 mm, typically 3 to 12 mm; a depth comprised within the range of 10 to 33 mm, typically within the range of 13 to 20 mm, such as 10,5 to 15,5mm.

The basin 26 may comprise a filling port 22, adapted to receive a reservoir.

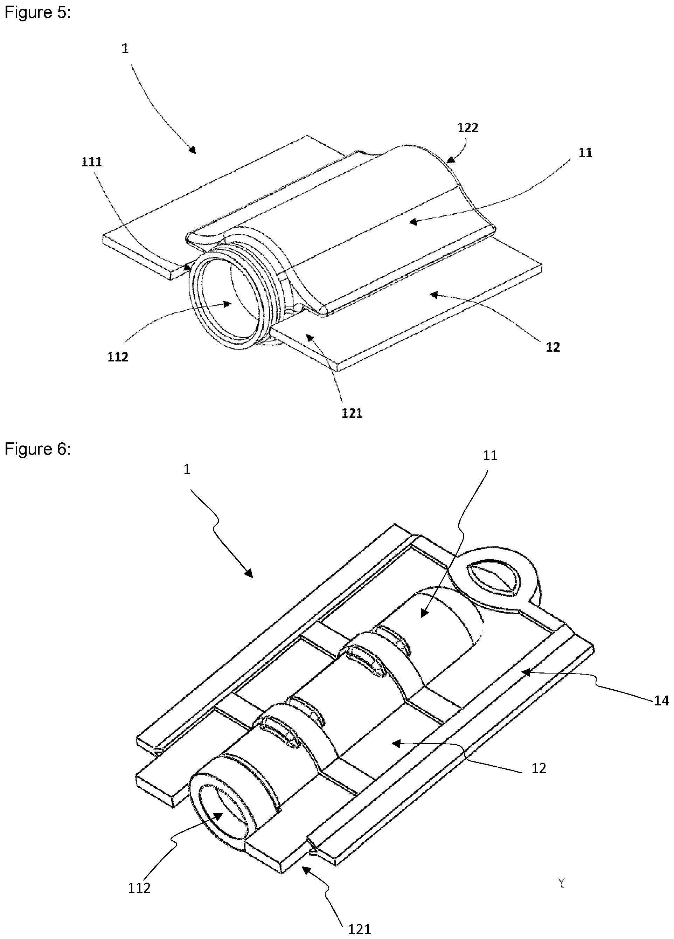

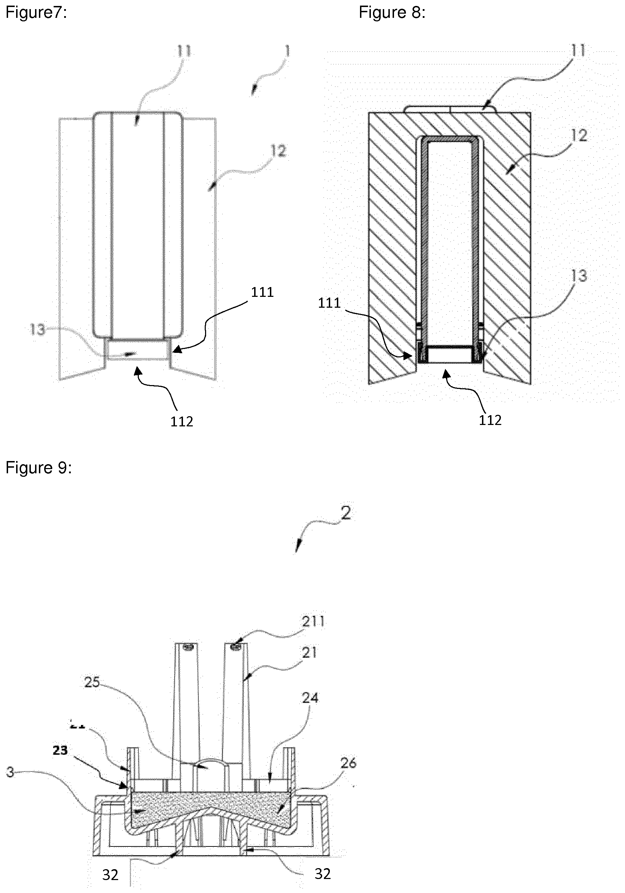

Figures 5 and 6 show perspective views of reservoirs according to two different embodiments, each being able to connect with the filing port 22 of the basin 26. The reservoirs 11 are each part of a cartridge 1. Fig 7 and 8 show respectively a side view and a cross-section view of the cartridge 1 according to the embodiment of figure 5

The reservoir 11 has a rupturable seal 13 and is filled with volatile liquid. To connect with the reservoir 11, the filling port 22 comprises a piercer 25 adapted to rupture the rupturable seal 13 of the reservoir 11 upon engagement of the reservoir 11 into the filing port 22. The engagement of the reservoir 11 is preferable made by plugging the reservoir 11 on the basin 26.

This piercer is located on the upper surface of the basin 26 and extends outside the basin 26 to come in contact with the reservoir 11. The piercer 25 leads the volatile liquid from the reservoir 11 to the interior cavity of the basin 26. The piercer may further comprise an inside extension as illustrated in the sectional view of Figure 4.

In such a proposed embodiment with a reservoir, the basin is an intermediate basin, the term "intermediate" indicating that it contains the volatile liquid to be dispersed by the dispensing material albeit supplied from the reservoir unit. In this proposed embodiment with an intermediate basin, the volume of the basin may be within the range of 1 to 6ml, preferably within the range of 1 to 4 ml.

According to an alternative embodiment, the volume of the basin may be sufficient to constitute its own reservoir. The volume of the basin may therefore be within the range of 6 to 60 ml, preferably within the range of 10 to 20 ml. In such an embodiment, the basin may be devoid of any filling port, or may comprise filling port for connecting with an additional reservoir.

Irrespective of the provision of a reservoir as a distinct part from the basin, the basin 26 is substantially closed to prevent the free dispersion of the volatile liquid but comprises at least one access opening. The access opening allows the access from the outside to the volatile liquid contained in the basin. The access opening may take the shape of a through slot 23 extending from one side to the other side of the filling port as illustrated in Figure 3. The access opening can take other shapes.

The access opening is typically provided to allow access to the volatile liquid by the use of a dispensing material.

Typically, the dispensing material 12 is a material which exhibits a porosity and capillarity to allow a volatile liquid to impregnate and travel along its inner structure.

Accordingly, another element of the proposed device is the dispensing material 12. The dispensing material 12 is adapted to be in fluid communication with the basin 26 through the access opening to allow the volatile liquid contained into the basin 26 to diffuse into the atmosphere. Therefore the liquid contained in the intermediate basin 26 is dispensed in the surrounding atmosphere by the dispensing material 12.

In order to prevent the free dispersion or the leakages of the volatile liquid in the event of a fall for example, it is proposed that the basin 26 includes an anti-dripping system.

The term "anti-dripping system" designates a system that prevents leakages. In this context, the anti-dripping system avoids dripping of the volatile liquid outside of the basin.

The anti-dripping system comprises an absorbing element. The Figure 9 shows the cross section view of Figure 4, with the absorbing element disposed in the basin. Further, the absorbing element 3 is disposed in the basin 26 by covering the access opening.

Accordingly, when trying to leak out of the basin, the volatile liquid contained within the basin 26 is first in contact with the absorbing element 3. The absorbing element thus holds the volatile liquid back in the basin. The volatile liquid is consequently restrained from further leaking out of the basin.

The absorbing element 3 is all the more stopping the volatile liquid form leaking out that the absorbing element may be porous. Porosity provides that absorbing element increases the surface tension of the volatile liquid contained therein. When the basin 26 is tilted or overturned, the increased surface tension helps to compensate the natural, i.e. gravitational, flow of the volatile liquid out of the basin.

Preferably, the absorbing element 3 fills the basin 26, more preferably the absorbing element 3 fills completely the basin. In another embodiment, the absorbing element does not fill completely the basin. Independently of filling or not the basin, the absorbing element 3 is preferably not extending outside of the basin, and stops at the access opening of the basin. In other words, the absorbing element 3 preferably extends up to the access opening of the basin.

The absorbing element 3 has appropriate dimension to fit in the intermediate basin and to be inserted at the bottom of the intermediate basin. The absorbing element 3 may be made of a porous material, typically a foam material, preferably a sponge, more preferably a synthetic or cellulosic sponge. The absorbing element 3 is preferably formed of a resilient material, notably elastically compressible and/or elastically extendable of at least 10%. According to the proposed device, the term "sponge" designates an absorbing material, porous and/ or fibrous, natural or synthetic, used to absorb a great quantity of liquid.

This anti-dripping system is simple and cost efficient by notably avoiding the use of a number of intermediate parts with low manufacturing tolerance as proposed in WO 2012/033526. Further, in the proposed device, the raw material of the absorbing element is inexpensive and abundant.

The porous material may also be hydrophilic to accelerate the impregnation of the volatile liquid in the sponge. It may be composed for example of hydrophilic polyurethane foam, open-pore foamed material, absorbent foam material, foam material based on a vinyl acetate polymer, cellulose-based material, this list not being limiting.

No clogging problems are expected with the absorbing element of the proposed device as the pores are big enough for the volatile liquid. The average pore diameter may nevertheless be less than 1 mm.

In particular the absorbing element 3 differs from the dispensing material 12. As above described, they notably differs in function. The absorbing element, absorbs and retains the volatile liquid, whereas the dispensing material absorbs and dispense the volatile liquid into the atmosphere. The retention of the absorbing element 3 is designed to retain the volatile liquid notably in case of a fall of the dispenser whereas the dispensing material 12 may not. Further the absorbing element 3 may notably differ structurally from the dispensing material 12. In particular, the absorbing element 3 may present a higher porosity. The absorbing element, e.g. of 54,5 cm² is typically capable to absorb 2,4g of volatile liquid in 40 minutes.

The proposed devices comprise different elements which all cooperate together to dispense the volatile liquid into the environment. The basin 26, the dispensing material 12 and the absorbing element 3 have been previously described. The basin contains the volatile liquid and the absorbing element 3 is responsible with the dispensing material 12 for the release of the volatile liquid into the atmosphere.

More specifically, the basin 26 may have different shapes, e.g. cubic, rectangular or circular. Also, as further exemplified in Figure 4, the basin does not need to have the same depth over the entire section.

The dispensing material 12 may have different forms and sizes. The dispensing material 12 is to be adapted to enter in fluid communication with the volatile liquid contained within the basin 26, for example by plunging into the volatile liquid of the basin 26. The dispensing material is preferably not directly in contact with the liquid contained within the basin 26 but rather gets in contact with the liquid through the absorbing element 3 of the dispensing device. In an alternative embodiment with the absorbing element not completely filling the basin 26, the dispensing material may be in direct contact with the liquid in the basin without the intermediary of the absorbing element 3. Accordingly the absorbing element may be provided with a slot (not shown) letting the dispensing material going through the absorbing element 3. In such an embodiment, the absorbing element is sufficiently resilient to abut against the crossing dispensing material without forming any leaking hole.

The size and overall surface of the dispensing material can be adapted to reach a specific rate of release of the volatile liquid and adapted to a given use. Typically, the higher is the surface of dispensing material 12 in contact with the atmosphere, the higher is the amount of volatile liquid released to the environment. For example, an appropriate surface of exchange of dispensing material 12 is comprised within the range of 2 000 to 10 000 mm², typically within the range of 3 000 to 7 000 mm² such as about 5 500 mm². A surface of exchange within the meaning of the proposed device should be construed as the surface of the dispensing material 12 outside the basin 26 which is in contact with the atmosphere once the supplying unit is placed on the basin 26.

In a preferred embodiment the dispenser may also comprise a supply unit. The supply unit may take the form of the cartridge 1 having a reservoir as previously discussed in reference to Figures 5 to 8. The reservoir 11 corresponds to an impermeable recipient which is adapted to contain a volatile liquid. Appropriate reservoirs have a capacity comprised within the range of 3 to 100 ml, typically within the range of 6 to 60 ml, preferably within the range of 10 to 20 ml. The capability of the reservoir can be adapted to the time of dispersion of the volatile liquid contemplated with the system and/or the size of the basin used with the supplying unit. Typically, the reservoir envisaged in the proposed device comprises a neck 111 and one opening 112. The opening 112 is further materialized in figures 7 and 8 by the rupturable seal 13. Notably the reservoir corresponds to a bottle as it appears in the Figures 5 and 6. Such bottles are easily manufactured which is convenient for the overall manufacture of the dispensing device. During storage the reservoir is closed to isolate the volatile liquid from the exterior atmosphere and prevent its vaporization.

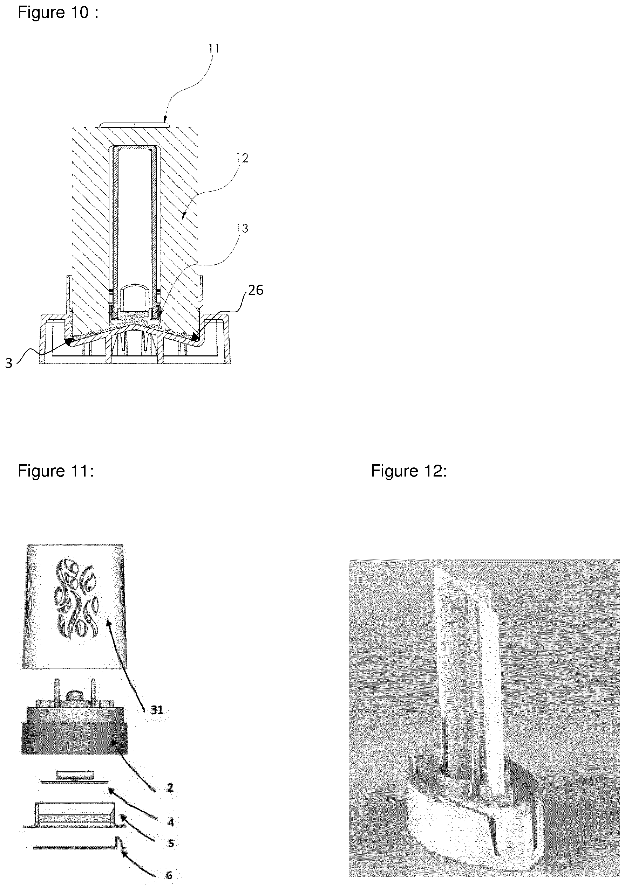

The reservoir is configured to open when it is plugged on the filling port 22 of the basin 26 as previously discussed. Typically, the reservoir 11 is placed with the opening 112 facing downwards onto the external extension of the piercer 25 of the basin 26, as illustrated in Figure 10. The piercer 25 has a size which fits the size and form of the opening 112 of the reservoir 11 and ruptures the seal 13 to release the volatile liquid into the basin 26 while maintaining the reservoir 11 into the same position. The basin is then supplied by gravity with the volatile liquid. As shown in Figures 4 and 10, the basin 26 may comprise reinforcing means 32 to support the pressure during the plugging of the reservoir 11 on the filing port so as to break the rupturable seal 13. A lip 24 is also present to close merely entirely the basin except for the access opening allowing the connection between the cartridge 1 and the base 2.

When a volatile liquid is supplied to the basin 26, for example from the reservoir 11, it fills the basin 26, get absorbed by the absorbing element 3. One additional advantage of the absorbing element 3 is that the level of liquid within the basin 26 remains constant. The amount of volatile liquid which is released into the atmosphere is constantly compensated by gravity from the reservoir 11 until it eventually gets empty. Indeed, the dispensing material 12 plunges inside the basin 26 in contact with the absorbing element 3, the volatile liquid rises by capillarity along the dispensing material 12 of the dispensing strip until it gets in contact with the surrounding atmosphere where it is released.

This aspect of the invention is particularly advantageous in comparison to products which are deprived of fixed reservoir where the basin needs to be refilled with volatile liquid on a regular basis. When the amount of liquid contained within the basin decreases, the amount of liquid which remains in contact with the dispensing material also decreases which eventually affects its capillarity capability and affects the rate of release of the volatile liquid to the atmosphere. The instant invention overcomes this problem by ensuring a constant rate of dispersion of volatile liquid, since the level of liquid is kept constant thanks to the absorbing element 3.

The continuous emission of the volatile materials with the devices of the invention can be of any suitable length, including but not limited to, up to: 20 days, 30 days, 60 days, 90 days, shorter or longer periods, or any period between 30 to 90 days. The duration of the emission will depend on the amount of liquid volatile supplied to the device and/or the surface of the dispensing material. It can be adapted according to the contemplated use and/or location.

Further, the absorbing element 3 also provides that even when the reservoir 11 is empty, volatile liquid keeps diffusing into the atmosphere. Indeed the absorbing element 3 always retains a little portion of the volatile liquid providing a remaining diffusion of the volatile liquid. When the reservoir 11 gets empty, a new reservoir can then be replaced and adapted to the basin to supply a new amount of volatile liquid to the dispensing device.

Notably as shown in Figures 5 to 8, it is proposed that the dispensing material 12 and the reservoir 11 filled with volatile liquid are forming a single part, i.e. the cartridge 1. Such a cartridge forms a removable and disposable part that can be replaced independently from the rest of the element of the dispensing device. For example, the dispensing material 12 is in the form of at least one strip which is coupled to the reservoir 11. When several strips are used, these are arranged radially from the outer surface of the reservoir, preferably as part of the surface which corresponds to the symmetry plan of the reservoir.

In reference to the embodiment illustrated in Figure 5, the dispensing material 12 is coupled on both sides of the reservoir, within the plan of symmetry of the specific form of said reservoir 11.

According to the proposed device, the dispensing material 12 may further cover all the outer surface of the reservoir or may comprise leave one portion free to let the user size the reservoir with his fingers without any risk of chemical contamination and without touching the wet impregnated dispensing material, i.e. when the supplying unit is empty.

According to a preferred embodiment, the dispensing material 12 is coupled to the outer surface of the reservoir 11 with an intermediate element which maintains together the two means. For example, as illustrated in Figure 6, a frame 14 may hold together the strips of the dispensing material 12 onto the outer surface of the reservoir 11. The term "frame" within the meaning of the invention encompasses a cage or any element which at least surrounds the periphery of the dispensing material.

The frame 14 may further be used as a guide to direct the cartridge 1 towards a given location on the basin 26 so that the opening 112 of the reservoir 11 gets in line with the piercer 25 of the basin 26 and remains straight during use. It also serves as a supporting mean to maintain the cartridge 1 in a given position. Depending on the design of the reservoir 11 and the position of its opening 112, the frame 14 keeps the supply unit 1 either inclined or vertically straight, to ensure that the liquid contained in the reservoir keeps flowing inside the basin. A vertical position is preferred to prevent any loss of liquid inside the reservoir during use. The term "vertically straight" means a position which forms substantially a 90° angle with the basis of the basin.

Typically, the frame 14 may cooperate and be inserted in rails 21 dispersed around the basin 26 when it is placed on it, as illustrated in Figures 4 and 9. Some rails 21 may correspond to upside protrusions of the through slot 23. The frame is guided by the rails 21 and maintained in a vertically straight position onto the basin while bringing the dispensing material 12 in contact with the volatile liquid contained within the basin 26 through the appropriate through slot 23.

Other guiding means placed either on the basin or the frame of the supply unit can be envisaged within the instant invention such as a clip or a prominent element.

Alternatively the dispensing material 12 may be directly coupled to the outer surface of the reservoir 11, e.g. with glue or any other suitable means.

When operating the above-described dispensing device with a dispensing material coupled with the reservoir, the user, with a single step, plug the reservoir 11 into the filling port 22 of the device, making the volatile liquid therein into fluid communication with the basin 26 of the device. It is preferred that the reservoir is compressing the absorbing element 3 into the basin 26 as illustrated in Figure 10. This compression is lowering the size of the pores of the absorbing element 3; enhancing thus the retention effect of the volatile liquid. The compression is also facilitating the diffusion of the volatile liquid into the atmosphere.

Because the dispensing material is coupled with the reservoir, when plugging the reservoir, the user is also placing the dispensing material 12 of the device into fluid communication with the basin 26 through the access opening; preferably entering in contact with the absorbing material 3 therein. The dispensing material 12 may comprise a portion 121 extending below the opening section of the reservoir to be able to plunge below the level of liquid of the basin into the liquid. According to another embodiment, the dispensing material 12 does not extend only over the entire height of the reservoir but extends below its opening section. Another appropriate design corresponds to an asymmetric dispensing material, where several strips are coupled to the outer surface of the reservoir and connected between them while only one strip extends below the opening section to reach the liquid. According to another embodiment, the dispensing material 12 does not extend beyond the upper extremity 122 of the reservoir 11.

The construction of the device in two parts, i.e. a basin on the one hand and the above-discussed cartridge in the other hand, provides an advantageous way to balance the lifetime of the dispensing material 12 with the amount of volatile liquid supplied to the system. When the reservoir needs to be replaced, a new and clean dispensing material 12 comes with the new supply unit; facilitating thus the manufacture of the supply unit. The construction of the device in two parts also increases the lifetime of the dispensing material 12 in a way that the dispensing material 12 is not soaked with the liquid before the cartridge is actually operated. It further prevents the product from being activated before use during storage.

The construction of the device in two parts also prevents clogging problems, usually encountered with the dispensing systems that use the same piece of material to disperse the volatile liquid throughout the entire lifetime of the device or when the user cannot correctly assess when it is the best time to replace the material.

An example of an embodiment of the proposed device is shown in Figure 11.

As illustrated in Figure 11, a housing 31 also designated as a cap may further be placed onto the dispensing device, i.e. to suit and mask the dispensing device. Accordingly the housing 31 may have a decorative function. The housing may notably be a display housing for displaying e.g. a picture printed on the housing, or a picture inserted on the housing. The picture printed or inserted on the housing may notably be customized by the user. The housing or cap can comprise holes to let the volatile liquid pass through the housing cap to the environment. Accordingly the housing or cap may only partially surround and mask the dispensing device. Alternatively the housing or cap may totally, at least laterally, surround the dispensing device. The housing is preferably made of paper but it can be made of different material and it can take other shapes. Typically, the housing 31 may cooperate and be inserted in a guiding slot 27 located around the basin 26 in order to be placed on it, as illustrated in Figure 3.The housing is guided by the guiding slot 27 and holding means 28; maintaining it in a vertically straight position onto the base 2.

As shown in the embodiment of Figure 11, the instant invention may comprise a heating element 4. The heating element 4 is designed to heat the basin, as well as the liquid inside the basin 26. The heating element 4 is powered by an autonomous system. The heating element does not require to be connected to a central electrical system to operate as would be any "plug-in" devices know in the art. Without being limitative, the power source may be any of a battery system, a solar cell system and the like. Other appropriate systems may be batteries which are regenerated by regular mean, typically standard AAA/AA battery charger, or by induction with a generator placed in a furniture such as a table.

In reference to the embodiment illustrated in Figure 11, the different components of the preferred embodiment for the proposed device according are represented. First, the cap 31 may be placed onto the dispensing device; more precisely onto the base 2 and the supply unit (not represented). Underneath the base 2 is placed the heating element 4 which comprises any type of heating systems that could fit into the dispenser. The heating element 4 forces the volatile liquid to evaporate from the base 2. Then, a battery case 5 may be placed underneath the heating element 4 in order to shelter the batteries necessary to operate the invention. Finally, a battery case lid 6 may be placed underneath the battery case 5 to protect the batteries and to close the battery case 5.

By "heating system" it is understood an apparatus which generates heat to be transmitted to the volatile liquid contained into the intermediate basin. The thus generated heat forces the volatile composition to evaporate directly from the basin to the surrounding areas.

Examples of appropriate heating element correspond to light bulbs or any type of resistors.

Another aspect of the proposed device is concerned with the process for dispensing volatile liquid according to the invention. In addition, the proposed process has been advantageously simplified in comparison to the existing dispensers.

Although limited embodiments of dispensing device and its components have been specifically described and illustrated herein, many modifications and variations will be apparent to those skilled in the art. For example, the device of the proposed device has been described to be suitable for dispensing fragrance. But the device is suitable for purposes of providing air fresheners, deodorizers, odor eliminators, malodor counteractants, insecticides, insect repellants, medicinal substances, disinfectants, sanitizers, mood enhancers, and aromatherapy aids, or for any other purpose using a volatile material that acts to condition, modify, or otherwise change the atmosphere or the environment.

Further, suitable materials for the dispensing material encompass wood, ceramics, terracotta paper, cotton, synthetic fiber, porous plastic and the like, preferably synthetic fibers. Other materials may be envisaged within the scope of the invention.

Furthermore, the dimensions of the proposed dispensing device are preferably chosen so that the volatile liquid dispensing device may be used in different locations, e.g. in a house or other confined spaces. Accordingly, the proposed device is preferably not cumbersome and may be hold in one hand. Such a proposed device does not have to be hanged and can advantageously be left on a piece of furniture.

## Claims

1. A dispensing device comprising
- a basin (26) adapted to contain a volatile liquid to be diffused into the atmosphere, the basin (26) containing an access opening from the outside to the volatile liquid contained therein;
- a dispensing material (12) adapted to be in fluid communication with the basin (26) through the access opening to allow the volatile liquid to diffuse into the atmosphere;
**characterised by**
the basin (26) including an anti-dripping system comprising an absorbing element (3); and the absorbing element (3) being disposed in the basin (26) by covering the access opening, filling the basin and extending up to the access opening.

2. The dispensing device according to claim 1, wherein the absorbing element (3) is made of a porous material.

3. The dispensing device according to claim 2, wherein the porous material is a foam material, preferably a sponge, more preferably a synthetic or cellulosic sponge.

4. The dispensing device according to any of claims 1 to 3, wherein the dispensing device comprises the volatile liquid inside the basin (26), the volatile liquid being a fragrance.

5. The dispensing device according to any of claims 1 to 4, comprising a base (2) including the basin (26), the base (2) further comprising a heating element (4) for heating the basin and, if necessary, the liquid inside the basin, (26), the heating element (4) being preferably at least one light bulb or at least one resistor.

6. The dispensing device according to any of claims 1 to 5, wherein the basin (26) is an intermediate basin and the dispensing device comprises a filling port (22) adapted to receive a reservoir (11) having a rupturable seal (13) and filled with volatile liquid, the filling port (22) comprising a piercer (25) for rupturing the rupturable seal (13) upon engagement of the reservoir (11) into the filling port (22), the dispensing device preferably including the reservoir (11).

7. The dispensing device according to claim 6, wherein the dispensing device comprises a cartridge (1) including the dispensing material (12) and the reservoir (11), the cartridge further comprising a coupling element (14) holding together the reservoir (11) and the dispensing material (12).

8. The dispensing device according to claim 6 or 7, wherein the access opening is in the form of a through slot (23) extending from one side to the other of the filling port.

9. The dispensing device according to any of claims 1 to 8, comprising a display housing (31), preferably made of paper, and being able to be placed onto the dispensing device in order to surround and mask, at least partially, the dispensing device.

10. A process for dispensing volatile liquid comprising the steps of :
- providing a device according to anyone of claims 6 to 9,
- plugging the reservoir (11) into the filling port of the device, thereby making the volatile liquid therein into fluid communication with the basin (26) of the device;
- placing the dispensing material (12) of the device into fluid communication with the basin (26) through the access opening.

11. The process according to claim 10, wherein the dispensing material (12) is placed into fluid communication with the basin (26) by contacting the absorbing element (3).

12. The process according to claim 10 or 11, wherein the reservoir (11) plugged into the filling port is compressing the absorbing element (3) into the basin.

13. The process according to any of claims 10 to 12, wherein the dispensing material is placed into fluid communication with the basin (26) by plugging the reservoir (11) into the filling port of the device.

## Patentansprüche

1. Ausgabevorrichtung, umfassend
- ein Becken (26), das ausgelegt ist, um eine flüchtige Flüssigkeit zu enthalten, um in die Atmosphäre diffundiert zu werden, wobei das Becken (26) eine Zugangsöffnung von der Außenseite zur flüchtigen Flüssigkeit, die darin enthalten ist, enthält,
- ein Ausgabematerial (12), das ausgelegt ist, um in fluidischer Kommunikation mit dem Becken (26) durch die Zugangsöffnung zu sein, um zu ermöglichen, dass die flüchtige Flüssigkeit in die Atmosphäre diffundiert; **dadurch gekennzeichnet, dass**
das Becken (26) ein Tropfschutzsystem einschließt, das ein absorbierendes Element (3) umfasst, und wobei das absorbierende Element (3) im Becken (26) angeordnet ist, indem es die Zugangsöffnung abdeckt, das Becken füllt und sich nach oben zur Zugangsöffnung erstreckt.

2. Ausgabevorrichtung nach Anspruch 1, wobei das absorbierende Element (3) aus einem porösen Material hergestellt ist.

3. Ausgabevorrichtung nach Anspruch 2, wobei das poröse Material ein Schaummaterial ist, vorzugsweise ein Schwamm, insbesondere ein synthetischer oder Zelluloseschwamm.

4. Ausgabevorrichtung nach einem der Ansprüche 1 bis 3, wobei die Ausgabevorrichtung die flüchtige Flüssigkeit innerhalb des Beckens (26) umfasst, wobei die flüchtige Flüssigkeit ein Duftstoff ist.

5. Ausgabevorrichtung nach einem der Ansprüche 1 bis 4, umfassend eine Basis (2), darin eingeschlossen das Becken (26), wobei die Basis (2) weiter ein Heizelement (4) zum Erhitzen des Beckens und, falls erforderlich, der Flüssigkeit innerhalb des Beckens (26) umfasst, wobei das Heizelement (4) vorzugsweise mindestens eine Glühbirne oder mindestens ein Widerstand ist.

6. Ausgabevorrichtung nach einem der Ansprüche 1 bis 5, wobei das Becken (26) ein Zwischenbecken ist und die Ausgabevorrichtung einen Befüllanschluss (22) umfasst, der ausgelegt ist, um einen Behälter (11) mit einer zerbrechbaren Dichtung (13) aufzunehmen, und mit flüchtiger Flüssigkeit gefüllt ist, wobei der Befüllanschluss (22) eine Einstechvorrichtung (25) zum Brechen der zerbrechbaren Dichtung (13) beim Eingriff des Behälters (11) in den Befüllanschluss (22) umfasst, wobei die Ausgabevorrichtung vorzugsweise den Behälter (11) einschließt.

7. Ausgabevorrichtung nach Anspruch 6, wobei die Ausgabevorrichtung eine Patrone (1) umfasst, darin eingeschlossen das Ausgabematerial (12) und den Behälter (11), wobei die Patrone weiter ein Kopplungselement (14) umfasst, das den Behälter (11) und das Ausgabematerial (12) zusammenhält.

8. Ausgabevorrichtung nach Anspruch 6 oder 7, wobei die Zugangsöffnung die Form eines Durchgangsschlitzes (23) aufweist, der sich von einer Seite zur anderen des Befüllanschlusses erstreckt.

9. Ausgabevorrichtung nach einem der Ansprüche 1 bis 8, umfassend ein Anzeigegehäuse (31), vorzugsweise hergestellt aus Papier, das auf die Ausgabevorrichtung platziert werden kann, um die Ausgabevorrichtung mindestens teilweise zu umgeben und zu maskieren.

10. Verfahren zur Ausgabe von flüchtiger Flüssigkeit, umfassend die folgenden Schritte:
- Bereitstellen einer Vorrichtung nach einem der Ansprüche 6 bis 9,
- Einstecken des Behälters (11) in den Befüllanschluss der Vorrichtung, dadurch Erzeugen einer fluidischen Verbindung zwischen der flüchtigen Flüssigkeit darin und dem Becken (26) der Vorrichtung;
- Bringen des Ausgabematerials (12) der Vorrichtung in fluidische Kommunikation mit dem Becken (16) durch die Zugangsöffnung.

11. Verfahren nach Anspruch 10, wobei das Ausgabematerial (12) in fluidische Kommunikation mit dem Becken (26) durch In-Kontakt-Bringen des absorbierenden Elements (3) gebracht wird.

12. Verfahren nach Anspruch 10 oder 11, wobei der in den Befüllanschluss gesteckte Behälter (11) das absorbierende Element (3) in das Becken komprimiert.

13. Verfahren nach einem der Ansprüche 10 bis 12, wobei das Ausgabematerial in fluidische Kommunikation mit dem Becken (26) durch Einstecken des Behälters (11) in den Befüllanschluss der Vorrichtung gebracht wird.

## Revendications

1. Dispositif de distribution comprenant :
- une cuvette (26) adaptée pour contenir un liquide volatil à diffuser dans l'atmosphère, la cuvette (26) contenant une ouverture d'accès à l'extérieur pour le liquide volatil contenu à l'intérieur de cette dernière ;
- un matériau de distribution (12) adapté pour être en communication fluide avec la cuvette (26) par le biais de l'ouverture d'accès pour permettre au liquide volatil de se diffuser dans l'atmosphère ;
**caractérisé en ce que** :
la cuvette (26) comprend un système anti-goutte comprenant un élément absorbant (3) ; et l'élément absorbant (3) est disposé dans la cuvette (26) en recouvrant l'ouverture d'accès, en remplissant la cuvette et en s'étendant jusqu'à l'ouverture d'accès.

2. Dispositif de distribution selon la revendication 1, dans lequel l'élément absorbant (3) est réalisé avec un matériau poreux.

3. Dispositif de distribution selon la revendication 2, dans lequel le matériau poreux est un matériau en mousse, de préférence une éponge, plus préférentiellement une éponge synthétique ou cellulosique.

4. Dispositif de distribution selon l'une quelconque des revendications 1 à 3, dans lequel le dispositif de distribution comprend le liquide volatil à l'intérieur de la cuvette (26), le liquide volatil étant une fragrance.

5. Dispositif de distribution selon l'une quelconque des revendications 1 à 4, comprenant une base (2) comprenant la cuvette (26), la base (2) comprenant en outre un élément de chauffage (4) pour chauffer la cuvette et, si nécessaire, le liquide à l'intérieur de la cuvette (26), l'élément de chauffage (4) étant de préférence au moins une ampoule ou au moins une résistance.

6. Dispositif de distribution selon l'une quelconque des revendications 1 à 5, dans lequel la cuvette (26) est une cuvette intermédiaire et le dispositif de distribution comprend un orifice de remplissage (22) adapté pour recevoir un réservoir (11) ayant un joint d'étanchéité pouvant être fracturé (13) et rempli avec le liquide volatil, l'orifice de remplissage (22) comprend un dispositif de perçage (25) pour fracturer le joint d'étanchéité pouvant être fracturé (13) suite à la mise en prise du réservoir (11) dans l'orifice de remplissage (22), le dispositif de distribution comprenant de préférence le réservoir (11).

7. Dispositif de distribution selon la revendication 6, dans lequel le dispositif de distribution comprend une cartouche (1) comprenant le matériau de distribution (12) et le réservoir (11), la cartouche comprenant en outre un élément de couplage (14) maintenant ensemble le réservoir (11) et le matériau de distribution (12).

8. Dispositif de distribution selon la revendication 6 ou 7, dans lequel l'ouverture d'accès se présente sous la forme d'une fente débouchante (23) s'étendant d'un côté à l'autre de l'orifice de remplissage.

9. Dispositif de distribution selon l'une quelconque des revendications 1 à 8, comprenant un boîtier d'affichage (31), de préférence réalisé à partir de papier et pouvant être placé sur le dispositif de distribution afin d'entourer et de masquer, au moins partiellement, le dispositif de distribution.

10. Procédé pour distribuer un liquide volatil comprenant les étapes suivantes :
- fournir un dispositif selon l'une quelconque des revendications 6 à 9,
- insérer le réservoir (11) dans l'orifice de remplissage du dispositif, permettant ainsi au liquide volatil d'être en communication fluide avec la cuvette (26) du dispositif;
- placer le matériau de distribution (12) du dispositif en communication fluide avec la cuvette (26) par l'ouverture d'accès.

11. Procédé selon la revendication 10, dans lequel le matériau de distribution (12) est placé en communication fluide avec la cuvette (26) en étant en contact avec l'élément absorbant (3).

12. Procédé selon la revendication 10 ou 11, dans lequel le réservoir (11) inséré dans l'orifice de remplissage comprime l'élément absorbant (3) dans la cuvette.

13. Procédé selon l'une quelconque des revendications 10 à 12, dans lequel le matériau de distribution est placé en communication fluide avec la cuvette (26) en insérant le réservoir (11) dans l'orifice de remplissage du dispositif.
